# EUROPEAN PATENT SPECIFICATION

(11) **EP 1 830 706 B1**
(45) Date of publication and mention of the grant of the patent: **21.07.2010**
(21) Application number: 05849506.0
(22) Date of filing: 14.12.2005
(51) Int. Cl.: A61B 5/107, A61M 25/01

(54) **MINIMALLY INVASIVE MEDICAL DEVICE WITH HELICAL PATTERN FOR INDICATING DISTANCE OF MOVEMENT**
MINIMAL INVASIVES MEDIZINPRODUKT MIT SPIRALFÖRMIGEM MUSTER ZUR ANGABE DER BEWEGUNGSDISTANZ
DISPOSITIF MEDICAL TRES PEU INVASIF AVEC MODELE HELICOIDAL POUR INDIQUER UNE DISTANCE DE MOUVEMENT

(30) Priority: 15.12.2004 US 636187 P
(43) Date of publication of application: 12.09.2007
(73) Proprietor: Wilson-Cook Medical Inc., Winston-Salem, North Carolina 27105 (US)
(72) Inventor: KENNEDY, Kenneth, C., II, Clemmons, North Carolina 27012 (US); HARDIN, David, M., Jr., Winston-salem, North Carolina 27106 (US)
(74) Representative: Garratt, Peter Douglas
(86) International application number: PCT/US2005/045253
(87) International publication number: WO 2006/065909

(56) References cited:
- EP-A- 1 348 461
- DE-A1- 19 823 414
- US-A- 4 951 686
- US-A- 5 379 779
- US-B1- 6 613 002
- US-B1- 6 796 976

## Description

### TECHNICAL FIELD

This invention relates to minimally invasive medical devices and particularly to wire guides and related devices.

### BACKGROUND

It has been recognized that the placement of a series of markings on a surgical instrument such as a wire guide, catheter, needle, etc., can aid a physician in proper placement of the device in the body of a patient during a medical procedure. These markings can include bands, dots, lettering, numbering, colors, or other types of indicia to indicate position or movement of the device within the body. Visually distinguishable marks are often located at regular predetermined intervals, e.g., placement of one dot or circumferential band at the 5 cm mark, two dots or circumferential bands at 10 cm, etc. Such a system of indicia can be made to be visible under fluoroscopy by the use of certain radio-opaque metals, or compounds incorporated into or printed on the device. When direct visualization is possible, numerical values imprinted on the device can be used as a scale for measuring structures or distance. The ability to quantify distances or make measurements is the primary benefit that has inspired the development of marker systems for wire guides, catheters, and the like.

Another use of markings is to provide a system whereby the clinician can determine relative movement of the device within the body. This has also been found to be useful in endoscopic procedures in which it is important to maintain a device at a stationary position or to detect relative movement of another component. One example is the use of an exchange wire guide, which has a tendency to become displaced as catheters or other instruments are advanced or withdrawn over the wire guide. When these procedures are performed using an endoscope, the wire guide can be visualized, and as a consequence, a pattern of markings configured to determine movement of the wire guide relative to the tip of the endoscope can help in restoring or maintaining proper position of the device.

In current wire guides, spiral or helical markings have been used for general determination of whether a medical device is being moved proximally or distally and - in combination with another pattern such as circumferential stripes - for determination of movement past a given point. However, such systems typically do not have a standardized helical color pattern width and therefore do not permit quantifying the amount of movement or making measurements within the body such as measuring the length of a stricture or lesion within a duct or vessel.

Direct visualization of indicia on the exchange wire guide via an endoscope offers some advantages; however, during procedures that access certain treatment sites such as the Papilla of Vater, mucous and other material can obscure the view, making direct measurement impossible. In addition, a prior art exchange wire guide that is useful for measuring strictures for quantifying distance can be inadequate for discerning movement when the visible area of the wire guide under endoscopy corresponds to gaps between markings. A system that permits endoscopic monitoring of the position of a wire guide to allow reliable and accurate measurement of anatomical structures is needed. Another desirable feature would be to combine the accurate measurement capabilities with a system that allows reliable detection of device movement during a medical procedure to assist in maintaining the device in a stationary position. Furthermore, it is desirable to be able to measure advancement of wire guides or other minimally invasive devices in procedures where the device is not visualized within the body, such as during certain endovascular procedures.

Document EP-A-1 348 461 discloses a guidewire to be inserted in a living body by way of an endoscope and comprises a visible mather having a spiral shape.

### BRIEF SUMMARY

The present invention provides a minimally invasive medical device, such as a wire guide, catheter, or sphincterotome, having an easy-to-use visual indicia system for monitoring distance of advancement. The visual indicia system includes a multi-colored or multi-patterned helix, or a series of colored/patterned dots, on the minimally invasive medical device and having a regular interval in the color/pattern so that the distance between the stripes on the helix (or between the different dots) indicates the distance of advancement. One advantage of the visual indicia system of the present invention is that minimally invasive medical devices may use a system of different colors and/or patterns to indicate total length and/or to indicate the widths of the helical stripes or the sizes of (and increment between) the dots useful in determining incremental advancement.

The foregoing problems are solved and a technical advance is achieved in an illustrative embodiment of an exchange wire guide, for use with an endoscope, having a helix or other indicia for indicating position and/or movement within a body of a patient. In the present invention, the elongate member (e.g., a standard solid nitinol core, polymer-coated exchange wire guide with a tapered or coil wire tip; a catheter; or some other medical device), includes a multi-colored helical indicia pattern that is at least partially visible by direct or endoscopic observation. The indicia pattern includes at least two differently colored bands helically oriented on the elongate member with the color bands sized such that the straight-line distance from an edge of a first occurrence of the first colored band to an edge of a second occurrence of the first colored band is a predetermined or standardized length increment. The present invention includes a method of using the multi-colored helical indicia to determine a distance within a body and a method of using the multi-colored helical indicia to determine the distance of proximal and/or distal movement of the elongate member at the proximal and/or distal end of an endoscope such as a duodenoscope.

When the indicia pattern of the present invention is used with an exchange wire guide, it permits the measurement of anatomical structures when used with an endoscope having an accessory channel for introducing ancillary devices or instrumentation. This obviates the need for separate measuring devices, and makes it especially useful for Endoscopic Retrograde Cholangiopancreatography (ERCP) procedures, which often requires the step of measuring the length of biliary strictures. Biliary strictures typically occur in the common bile duct. The common bile duct provides a path from the gall bladder to the duodenum. More specifically, the common bile duct proceeds from the junction of the common hepatic duct with cystic duct, which is open to the gall bladder, and merges with the pancreatic duct, forming the ampulla of Vater, which itself opens into the duodenum at the papilla of Vater. The sphincter of Oddi is a muscular ring that controls passage of fluid from the ampulla of Vater into the duodenum.

In one aspect of the invention specifically configured for an ERCP procedure, the endoscope is advanced into the duodenum to a point near the papilla of Vater. The sphincter of Oddi is cannulated (e.g., with a cannulating catheter, a sphincterotome, or a wire guide), and a wire guide is then advanced from the distal end of the endoscope into the papilla of Vater to access the biliary system. The tip of the endoscope preferably remains in the duodenum where visibility is superior and unobscured by mucous and bile. The bile duct is typically visualized by fluoroscopy wherein radio-opaque contrast fluid is introduced into the bile duct to provide an image of the ductal structure relative to the device (e.g., wire guide, sphincterotome, catheter) being used therein.

To measure a biliary stricture, a wire guide having a radio-opaque marker on the distal portion is advanced until it has crossed the stricture. The wire guide may be made fluoroscopically visible by loading the elastomeric material comprising the tip with a radio-opaque material, such as a tungsten or barium powder (e.g., by a method such as that disclosed in U.S. Pat. No. 5,300,048 to Drewes); by addition of a second radio-opaque material, such as applying radio-opaque bands, shrink tubing, or dipped material; by making the wire itself radio-opaque, such as by placement of a platinum coil over a tapered solid core wire; or by use of a radio-opaque wire guide core material, such as stainless steel.

In one example of how the present invention can be utilized, the clinician determines the position of the wire guide in a biliary duct using an endoscope, then withdraws the wire guide, counting the number of bands of one color that are exposed on the distal portion of the wire guide, until the radio-opaque tip marks the proximal boundary of the stricture. Alternatively, the number of helical bands may be counted at the proximal end of the endoscope where the wire guide protrudes from and is manipulated through an access port. The length associated with the helical bands on the wire guide permits the length of the stricture readily to be ascertained. This information can be important in subsequent treatment or procedures, such as determining the correct sizing of a biliary stent for placement across the stricture. Once the stricture has been measured, the wire guide can be maintained in place to serve as an exchange wire for introducing other instruments. Other applications of the present invention can be made in endovascular and other minimally invasive procedures.

### BRIEF DESCRIPTION OF THE DRAWINGS

Embodiments of the present invention are disclosed by way of example with reference to the accompanying drawings, in which:

FIG. 1 depicts a side view of the illustrative embodiment of the present invention;

FIG. 2 illustrates an enlarged pictorial view of a portion of the device of FIG. 1 being used with an endoscope;

FIG. 3 is a view of the device of FIG. 1 being used in vivo;

FIG. 4 shows a partially sectioned side view of an alternative embodiment of the device of FIG. 1;

FIG. 5 shows a partial side view of another alternative embodiment of the present invention; and

FIG. 6 shows a partial side view of yet another alternative embodiment of the present invention.

### DETAILED DESCRIPTION

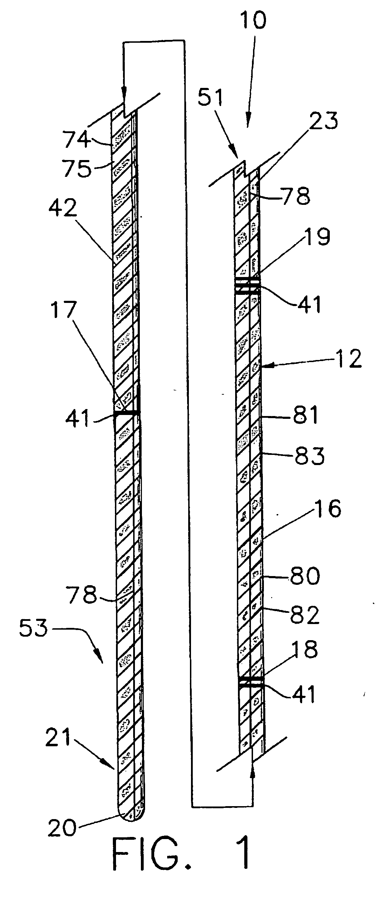

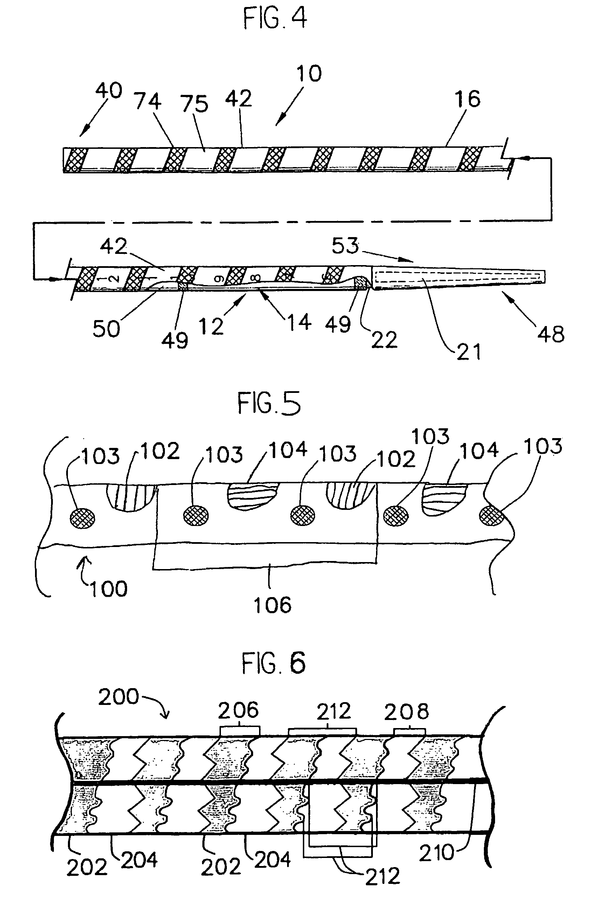

FIGS. 1-4 depict a minimally invasive medical device 10 comprising a wire guide 16 or similar elongate member having an indicia pattern comprising a multi-colored system of helical indicia 12 that allows the wire guide 16 to be used with an endoscope 25 to measure anatomical structures within a patient. The illustrative device 10 preferably comprises a standard exchange wire guide 16, e.g., 480 cm or 260 cm in length, with a solid core wire 50, such as nitinol, and an outer surface coating 42, such as PET or PTFE, that is shrink-wrapped over the wire (as is best shown in FIG. 4). To aid in fluoroscopic positioning of the wire guide 16, a distal portion of the device includes a marker material 21, either as a single marker, a plurality of markers, or an extended radio-opaque region that is several centimeters long (e.g., the distal 5 cm). Typical methods of providing radio-opacity include the addition of a distal platinum coil, adding gold or other radio-opaque material markers, using radio-opaque inks, or the use of radio-opaque shrink wrap or tubing over the core wire, e.g., radio-opaque urethane, or dipping the wire in a radio-opaque polymer, or affixing a polymer tip, such as PEBAX®, that has been loaded with radio-opaque powder, such as tungsten.

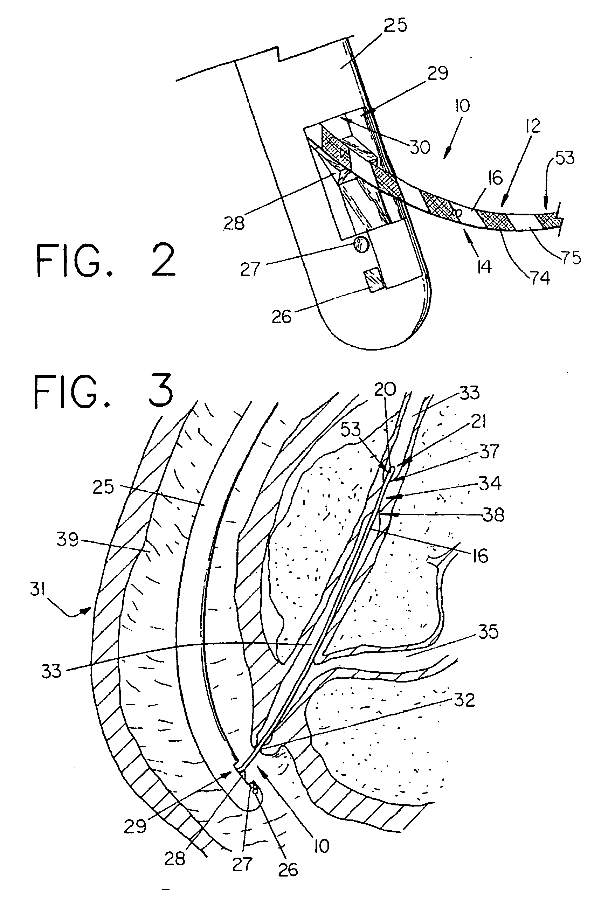

As shown in FIG. 2, the wire guide 16 includes a distal portion 53 designed to at least be partially advanced from the distal end of the accessory or working channel 30 of an endoscope, the distal portion 53 including the helical indicia pattern 12. An intermediate portion 51, shown in FIG. 1, lies between the proximal portion 40 (depicted in FIG. 4) and the distal portion 53. The intermediate portion 51, which may also include a portion of the helical indicia pattern 12, largely remains within the working channel of the endoscope during a majority of the medical procedure. The proximal portion 40 comprises the remainder of the wire guide 16 and is intended to substantially remain outside of the proximal end of the working channel of the endoscope. In the illustrative embodiment of FIG. 4, the proximal portion also includes a portion of the system of indicia 12, in the form of helical striping 74, 75, for helping the operator to better determine, via direct observation, the distance that the wire guide 16 is moving or has moved longitudinally. The stripes 74, 75 are marked in a manner that makes them distinguishable from each other (e.g. with different colors, patterns, or other distinctive markings). Optionally, the distinctive marking pattern (e.g., a selected color combination) can also be uniquely correlated to the diameter and/or length of the wire guide.

In the embodiments of FIGS. 1-4, the helical indicia pattern 12 includes a series of different colored helical striped markings 74, 75 of a predetermined or standardized width and/or spacing for permitting visual identification of the position of the wire guide 16 and quantifying distances of movement and lengths of anatomical structures. The width of these markings 74, 75 is determined by the pitch of the helices 74, 75 and corresponds to standard scale increments for measuring distance (e.g., increments of 1 cm, 5 cm, 1 mm, 0.1 in., etc.) such that the straight-line distance from each proximal edge of an occurrence of one color stripe to the next occurrence of the same color stripe, as measured parallel to the central longitudinal axis of the wire guide 16 is a predetermined, standard and/or consistent distance that is and is sufficiently uniform to allow use of the color stripe for measurement. Most preferably, the predetermined, standard and/or consistent distance that is and is sufficiently uniform to allow use of the color stripe for measurement is a standardized increment that is easily tracked by a user (e.g., increments of, for example, 0.5 mm, 0.1 cm, or 0.5 cm, rather than, for example, 3.14 mm or 0.338 cm).

Optionally, the markings 74, 75 of the system of indicia can include one or more unique series of non-helical markings such as circumferential bands 41 spaced at regular intervals along a portion of the device. The bands 41 are imprinted or applied to the wire guide as a separate material and can be radio-opaque (see FIG. 1). In the illustrative embodiments, the number bands may be increased (or decreased) to provide an additional second system of scale indicia to the helical indicia 74,75.

The helical markings may include more than the two helical stripe markings 74, 75. For example, three or more differently color helical stripes may be used so long as the width of each is predetermined, standardized, and/or substantially consistent so as to allow accurate measurements to be performed. Preferably, the colors or other marking patterns of the two or more helical stripes are selected from colors/patterns that are distinguishable in the various environments where a wire guide will be used. In particular, colors are preferably selected so that at least one or more of the marking patterns (e.g., helical stripes, circumferential bands, or other shaped markings) is visible in the conditions where the wire guide is to be used. For example, the marking pattern should be visible through an endoscope camera in the presence of bile, blood, or other bodily substances. One example of a color pattern for a three-color helically striped pattern includes the colors silver, orange, and black. In this example, at least two of the colors are visible under most conditions where the wire guide will need to be visualized. The visibility of the colors provides for an enhanced ability to use the color pattern for measurement.

As yet another option, and as illustrated in FIG. 1, one or more longitudinal lines 78 paralleling the central longitudinal axis of the wire guide 16 may be placed on the wire guide 16 exterior to aid in tracking and counting of the helical markings 74, 75. Importantly, the longitudinal line 78 allows the user to monitor or prevent any rotation of the wire guide 16 that could reduce the accuracy of the measurement.

In the illustrative embodiments, the helical scale indicia 12 for measuring movement are printed in ink on the outer coating 42 of the wire guide, although other well-known methods of imprinting or marking medical devices could be used. The outer coating 42 comprises a polymeric material such as PET or other suitable material. The outer coating 42 of portions 40, 51 and 53 of the wire guide may, however, comprise different materials. For example, the distal portion 53 can have a PET coating, which better adapted to receive printing, while the intermediate portion 51 and/or proximal portion 40 -on which no printing is required- can be made of PTFE (on which it is more difficult to print). If PTFE is used for the outer coating 42, it is advantageous to incorporate the color bands of the helical scale indicia 12 into the PTFE during application of the PTFE (by, for example, using a colored PTFE formulation) rather than attempting to print them onto the PTFE.

In an embodiment shown in FIG. 4, the distal-most portion of distal portion 53 of the device, e.g., the distal-most 5 cm of the wire guide 16, is advantageously made radio-opaque by gluing on or otherwise attaching a separate tapered tip 48 made of, for example, PEBAX® loaded with a core 21 comprising a radio-opaque powder such as tungsten. In this embodiment, the tapered tip 48 contains a shoulder 22 such that there is a smooth transition between the tip 48 and the outer polymeric coating 42, which then overlays the shoulder. The tapered tip 48 is glued to the core wire 50 (shown in FIG. 4) using cyanoacrylate or another suitable adhesive.

FIG. 4 also depicts an alternate type of indicia 12 that includes radio-opaque markers, such as radio-opaque tubing 49 (e.g., radio-opaque urethane), applied to the core wire 50 in the distal portion 53, with the coating 42 placed thereover. As depicted, the radio-opaque tubing 49 provides a series of radio-opaque indicia at regular intervals that correspond to a known scale for determining length (e.g., 5 cm intervals).

In the illustrative embodiment depicted in FIG. 1, the indicia pattern 12 on the distal portion 53 comprises a combination of two differently colored helical bands 74, 75. A first scale reference marking comprising a single band 17 is located 5 cm from the distal tip 20 of the wire guide 16, while a second reference marking, comprising two adjacent bands 18, is located 10 cm from the distal tip 20. A third reference marking, comprising three bands 19, is located 15 cm from the distal tip 20. Of course, the series could continue in this manner for a longer portion of the wire guide 16. As depicted in FIGS. 2 and 4, numeric indicia 14 are optionally located between the series of bands 17, 18, 19. Alternatively, these indicia optionally include non-unique intermediate reference marking, e.g., single circumferential lines at each 1 cm increment between the 5 cm bands. A system of numerical visual indicia is more fully described in U.S. Patent No. 6,613,002, assigned to Wilson-Cook Medical Inc., The straight-line distance between a selected point 80 on one occurrence (as a "helice step") of the stripe 74 and the selected point 82 on the next occurrence (as a "helice step") of the stripe 74 is a predetermined or standardized distance. Likewise, the straight-line distance between a selected point 81 on one occurrence (as a "helice step") of the stripe 75 and the selected point 83 on the next occurrence (as a "helice step") of the stripe 75 is a predetermined or standardized distance.

FIGS. 2-3 depict the wire guide 16 of the present invention being used with an endoscope 25. In FIG. 2, the wire guide 16 with the indicia pattern 12 is positioned at the desired point at which the optional numerical value 14 can be read via the camera lens 27 under illumination of the light source 26. If it is desired to maintain a steady position of the wire guide 16, such as during a catheter exchange procedure, the helical stripes 74, 75 provide a means to determine whether the wire is moving and in which direction.

As shown in FIG. 3, the illustrative wire guide embodiment is especially useful in an ERCP procedure to measure a stricture 34 in the common bile duct 33 or another site within the biliary system, such as the pancreatic duct 35. The endoscope 25 is first advanced down into the lumen 39 of the duodenum 31 until it nears the Papilla of Vater 32, which is the entrance to the biliary system from the duodenum 31. The wire guide 16 is then advanced from the accessory channel 30 of the endoscope 25 (see FIG. 2). The elevator 28 of the endoscope 25 is positioned to laterally deflect the wire guide 16 from the side opening 29 of the scope 25 to facilitate advancement through the duct 33 and toward the stricture 34. By utilizing the radio-opaque component 21 of the tapered tip 48 of the wire guide 16 (see FIG. 4), the device is guided under fluoroscopy to the distal point 37 of the stricture 34 (furthest from the Papilla of Vater 32). At that point, the user or endoscopist notes the color and alignment of the helical stripe 74 (or 75) relative to a visible point such as a point on the Papilla of Vater 32. Alternatively, a user may note the color and alignment of the helical stripe 74 (or75) on the proximal portion of the wire guide 16 relative to a visible point such as the point of the endoscope 25 nearest the wire guide 16. The wire guide 16 is withdrawn until the radio-opaque tip 21 corresponds to the proximal point 38 of the stricture 34 (closest to Papilla of Vater 32), counting the number of helical stripe 74 occurrences (i.e., the number of helical stripes 74 that have moved past the visual reference point) during withdrawal. The longitudinal line 78 can be used to monitor and/or ensure that the wire guide 16 does not rotate as it is being withdrawn, which could result in inaccurate measurement. At that time, the distance transited is calculated based on the number of helical stripe 74 (or 75) occurrences to determine the length of the stricture 34. Following the measurement, a catheter or other device can be advanced over the same wire guide 16 to thereby facilitate the exchange of different devices.

A second embodiment of the present invention of visual marking indicia is illustrated in FIG. 5 as a system of dots placed upon an elongate structure such as a wire guide 100. The dots are placed at standard intervals and are distinctively marked with a pattern or color that is visually distinguishable in the various environments where a wire guide is intended to be used. A first set of dots 102 has a first pattern/color, a second set of dots 103 has a second color/pattern, and a third set of dots 104 has a third color/pattern. The distance 106 between a point on one dot 102 and a corresponding point on the next adjacently spaced dot 102 having the same color/pattern is consistent and can be used in the same manner as the helix steps described above for determining, for example, the distance of movement of the wire guide or the length of a biliary stricture. In various embodiments, the dots 102, 103, 104 may be of different sizes and may be placed in a straight-line arrangement or some other arrangement (e.g., a staggered or spiraling arrangement) along the wire guide 100. In the embodiment illustrated, the dots 103 utilize a spacing that is approximately one half the spacing of either dots 102 or 104. Also, in various embodiments, the dots 102, 103, 104 may be used in conjunction with helical stripes. The dots 102, 103, 104 also provide the user with a means for determining and/or preventing rotation of the wire guide. The dots in the embodiment shown in FIG. 5 are circular, but in other alternative embodiments, the dots may have other visually distinguishable shapes and/or colors such as, for example, black squares, silver triangles, pink stars, yellow "X's", green clovers, and blue diamonds, or may be substituted with visually distinguishable circular bands. For example, in an alternative embodiment of an elongate device of the present invention (not shown), a set of visually distinctive markings include identical square dots placed along one longitudinal axis at 0.5 cm increments, identical circular dots placed along a parallel longitudinal axis at 1.0 cm increments, identical star-shaped dots placed along another parallel longitudinal axis at 2.0 cm increments, and identical circular bands marked along the device at 5 cm increments. In this embodiment, the placement of the visually distinctive markings provides for measurement and for monitoring and/or prevention of rotation of the device. In a further embodiment, this elongate device could also include helical markings at a pitch providing, for example a 1.5 cm increment.

FIG. 6 illustrates an alternative embodiment of a helical striping pattern of two stripes 202, 204 on a sphincterotome surface 200. The first stripe 202 includes a visually distinctive color or pattern that distinguishes it from the second stripe 204. The first stripe 202 has a first width 206 that is varied, and the second stripe 204 has a second width 208 that is also varied. The sphincterotome surface 200 includes a reference marking that is a lengthwise stripe 210 that extends parallel to the central axis of the sphincterotome. The varied-width stripes 202, 204 are useful in the same manner for measurement as described above because the sum of the first and second widths 206, 208 is substantially consistent. This sum is, in the illustrated embodiment, a predetermined width 212. The method of measuring a distance using this striping pattern is substantially the same as the method described above.

It should be understood that, although several of the illustrative embodiments include a wire guide having surface indicia, the indicia patterns described herein can be applied to any elongated minimally invasive medical device, such as a catheter, sphincterotome, or other related device, that is useful in a minimally invasive diagnostic or surgical procedure. The present invention may be used in other types of gastrointestinal procedures, as well as endovascular or other diagnostic and surgical environments where it is desirable to use minimally invasive surgical devices.

It is intended that the foregoing detailed description be regarded as illustrative rather than limiting, and that it be understood that it is the following claims, including all equivalents, that are intended to define the scope of this invention.

## Claims

1. A minimally invasive medical device for use with an endoscope (25), comprising:
an elongate shaft (16) comprising a helical pattern (12) at least partially visible by endoscopic observation of at least an alternating first distinctively marked band (74) having a first width and second distinctively marked band (75) having a second width
wherein, along a surface parallel to a central longitudinal axis of the shaft, a length interval between each of a plurality of occurrences of the first distinctively marked band is a known length increment and is sufficiently uniform to allow use of the minimally invasive medical device for measurement;
**characterized in that** the device further comprises
a reference marking (78) for ascertaining rotation of the shaft, the reference marking comprising a line extending parallel to the central longitudinal shaft axis.

2. The minimally invasive medical device of claim 1, wherein the distinctive marking of the first band is one of a color, a shape pattern, and a combination thereof disposed on the first band (74).

3. The minimally invasive medical device of claim 1, wherein the distinctive marking of the second band is one of a color, a shape pattern, and a combination thereof disposed on the second band (75).

4. The minimally invasive medical device of claim 1, wherein the distinctive marking of the first band (74) is different than the distinctive marking of the second band (75).

5. The minimally invasive medical device of claim 1, wherein at least one of the first width and the second widths comprises a varied width, and a sum of the first and second widths is substantially consistent.

6. The minimally invasive medical device of claim 1, wherein the first width is substantially different from the second width.

7. The minimally invasive medical device of claim 1, further comprising a third distinctively marked band having a third width and alternating with the first (74) and second (75) distinctively marked bands.

8. The minimally invasive medical device of claim 1, wherein the minimally invasive medical device is one of a wire guide, a catheter, and a sphincterotome.

9. The minimally invasive medical device of claim 1, wherein:
said first distinctively marked band (74) comprises at least a first set of identical individual first markings having a uniform first spacing therebetween and said second distinctively marked band (75) comprises a second set of identical individual second markings having a uniform second spacing therebetween.

10. The minimally invasive medical device of claim 9, wherein the first spacing is different than the second spacing.

11. The minimally invasive medical device of claim 9, further comprising a third set of identical individual third markings having a uniform third spacing therebetween and alternating with the first and second sets of markings.

12. The minimally invasive medical device of claim 9, wherein the first set of identical markings have a first distinctive visual appearance, and the second set of identical markings have a second distinctive visual appearance.

13. The minimally invasive medical device of claim 9, wherein each of the first and second distinctive visual appearance is selected from a group consisting of a distinctive shape, a distinctive color, and a combination thereof.

## Patentansprüche

1. Minimal invasives Medizinprodukt zur Verwendung mit einem Endoskop (25), das Folgendes umfasst: einen länglichen Schaft (16) mit einem spiralförmigen Muster (12), das durch endoskopische Beobachtung zumindest eines abwechselnden ersten unverwechselbar markierten Bands (74) mit einer ersten Breite und eines zweiten unverwechselbar markierten Bands (75) mit einer zweiten Breite zumindest teilweise sichtbar ist, worin über eine Fläche parallel zu einer zentralen Längsachse des Schafts ein Längenintervall zwischen jedem Erscheinen einer Vielzahl von Erscheinungen des ersten unverwechselbar markierten Bands ein bekanntes Längeninkrement ist und ausreichend gleichmäßig ist, um die Verwendung des minimal invasiven Medizinprodukts für Messungen zu gestatten; **dadurch gekennzeichnet, dass** das Produkt ferner eine Referenzmarkierung (78) zur Bestimmung der Drehung des Schafts umfasst, wobei die Referenzmarkierung eine Linie umfasst, die parallel zur zentralen Längsschaftachse verläuft.

2. Minimal invasives Medizinprodukt nach Anspruch 1, worin die unverwechselbare Markierung des ersten Bands eine auf dem ersten Band (74) angeordnete Farbe, ein Formmuster oder eine Kombination davon ist.

3. Minimal invasives Medizinprodukt nach Anspruch 1, worin die unverwechselbare Markierung des zweiten Bands eine auf dem zweiten Band (75) angeordnete Farbe, ein Formmuster oder eine Kombination davon ist.

4. Minimal invasives Medizinprodukt nach Anspruch 1, worin sich die unverwechselbare Markierung des ersten Bands (74) von der unverwechselbaren Markierung des zweiten Bands (75) unterscheidet.

5. Minimal invasives Medizinprodukt nach Anspruch 1, worin zumindest die erste Breite oder die zweite Breite eine variierende Breite umfasst und worin eine Summe der ersten und zweiten Breiten im Wesentlichen gleichbleibend ist.

6. Minimal invasives Medizinprodukt nach Anspruch 1, worin sich die erste Breite erheblich von der zweiten Breite unterscheidet.

7. Minimal invasives Medizinprodukt nach Anspruch 1, das ferner ein drittes unverwechselbar markiertes Band mit einer dritten Breite umfasst, das sich mit den ersten (74) und zweiten (75) unverwechselbar markierten Bändern abwechselt.

8. Minimal invasives Medizinprodukt nach Anspruch 1, worin das minimal invasive Medizinprodukt ein Führungsdraht, ein Katheter oder ein Sphinkterotom ist.

9. Minimal invasives Medizinprodukt nach Anspruch 1, worin: das erste unverwechselbar markierte Band (74) zumindest einen ersten Satz identischer individueller erster Markierungen mit einem gleichmäßigen ersten Abstand dazwischen umfasst und das zweite unverwechselbar markierte Band (75) einen zweiten Satz identischer individueller zweiter Markierungen mit einem gleichmäßigen zweiten Abstand dazwischen umfasst.

10. Minimal invasives Medizinprodukt nach Anspruch 9, worin sich der erste Abstand vom zweiten Abstand unterscheidet.

11. Minimal invasives Medizinprodukt nach Anspruch 9, das ferner einen dritten Satz identischer individueller dritter Markierungen mit einem gleichmäßigen dritten Abstand dazwischen umfasst, die sich mit dem ersten und zweiten Satz von Markierungen abwechseln.

12. Minimal invasives Medizinprodukt nach Anspruch 9, worin der erste Satz identischer Markierungen ein erstes unverwechselbares visuelles Aussehen aufweist und der zweite Satz identischer Markierungen ein zweites unverwechselbares visuelles Aussehen aufweist.

13. Minimal invasives Medizinprodukt nach Anspruch 9, worin das erste und zweite unverwechselbare visuelle Aussehen jeweils aus einer eine unverwechselbare Form, eine unverwechselbare Farbe oder eine Kombination davon umfassenden Gruppe ausgewählt ist.

## Revendications

1. Dispositif médical très peu invasif destiné à être utilisé avec un endoscope (25), comportant :
un arbre allongé (16) comportant un modèle hélicoïdal (12) au moins partiellement visible lors de l'observation endoscopique d'au moins une première bande alternée marquée de manière distinctive (74), d'une première largeur, et une deuxième bande marquée de manière distinctive (75) d'une deuxième largeur,
dans lequel, le long d'une surface parallèle à un axe longitudinal central de l'arbre, un intervalle de longueur entre chaque occurrence de la première bande marquée de manière distinctive parmi une pluralité d'occurrences est une augmentation connue de la longueur et est suffisamment uniforme pour permettre l'utilisation du dispositif médical très peu invasif à des fins de mesure ;
**caractérisé en ce que** le dispositif comporte en outre
un marquage de référence (78) pour déterminer la rotation de l'arbre, le marquage de référence comportant une ligne s'étendant parallèlement à l'axe longitudinal central de l'arbre.

2. Dispositif médical très peu invasif selon la revendication 1, dans lequel le marquage distinctif de la première bande est soit une couleur, un modèle de forme, ou une combinaison de ceux-ci disposés sur la première bande (74).

3. Dispositif médical très peu invasif selon la revendication 1, dans lequel le marquage distinctif de la deuxième bande est soit une couleur, un modèle de forme, ou une combinaison de ceux-ci disposés sur la deuxième bande (75).

4. Dispositif médical très peu invasif selon la revendication 1, dans lequel le marquage distinctif de la première bande (74) est différent du marquage distinctif de la deuxième bande (75).

5. Dispositif médical très peu invasif selon la revendication 1, dans lequel au moins une des première et deuxième largeurs comporte une largeur variée, et une somme des première et deuxième largeurs est sensiblement constante.

6. Dispositif médical très peu invasif selon la revendication 1, dans lequel la première largeur est sensiblement différente de la deuxième largeur.

7. Dispositif médical très peu invasif selon la revendication 1, comportant en outre une troisième bande marquée de manière distinctive, d'une troisième largeur, et alternant avec les première (74) et deuxième (75) bandes marquées de manière distinctive.

8. Dispositif médical très peu invasif selon la revendication 1, dans lequel le dispositif médical très peu invasif est un fil-guide, un cathéter, ou un sphinctérotome.

9. Dispositif médical très peu invasif selon la revendication 1, dans lequel :
ladite première bande marquée de manière distinctive (74) comporte au moins un premier ensemble de premiers marquages individuels identiques ayant un premier espace uniforme entre eux et ladite deuxième bande marquée de manière distinctive (75) comporte un deuxième ensemble de deuxièmes marquages individuels identiques ayant un deuxième espace uniforme entre eux.

10. Dispositif médical très peu invasif selon la revendication 9, dans lequel le premier espace est différent du deuxième espace.

11. Dispositif médical très peu invasif selon la revendication 9, comportant en outre un troisième ensemble de troisièmes marquages individuels identiques ayant un troisième espace uniforme entre eux et alternant avec les premier et deuxième ensembles de marquages.

12. Dispositif médical très peu invasif selon la revendication 9, dans lequel le premier ensemble de marquages identiques a un premier aspect visuel distinctif, et le deuxième ensemble de marquages identiques a un deuxième aspect visuel distinctif.

13. Dispositif médical très peu invasif selon la revendication 9, dans lequel chacun des premier et deuxième aspects visuels distinctifs est choisi dans un groupe constitué d'une forme distinctive, d'une couleur distinctive, et d'une combinaison de ceux-ci.
